# EUROPEAN PATENT SPECIFICATION

(11) **EP 3 735 477 B1**
(45) Date of publication and mention of the grant of the patent: **30.10.2024**
(21) Application number: 18897938.9
(22) Date of filing: 20.12.2018
(51) Int. Cl.: C12Q 1/6848

(54) **ENHANCEMENT OF NUCLEIC ACID POLYMERIZATION BY MINOR GROOVE BINDING MOIETIES**
ERHÖHUNG DER NUKLEINSÄURE-POLYMERISATION DURCH MINOR-GROOVE-BINDEEINHEITEN
AMÉLIORATION DE LA POLYMÉRISATION D'ACIDES NUCLÉIQUES PAR DES FRACTIONS DE LIAISON À UN PETIT SILLON

(30) Priority: 05.01.2018 US 201862614114 P
(43) Date of publication of application: 11.11.2020
(73) Proprietor: Roche Diagnostics GmbH, 68305 Mannheim (DE); F. Hoffmann-La Roche AG, 4070 Basel (CH)
(72) Inventor: KOKORIS, Mark Stamatios, Bothell, Washington 98011 (US); TABONE, John, Kirkland, Washington 98034 (US); NABAVI, Melud, Seattle, Washington 98109 (US); JACOBS, Aaron, Seattle, Washington 98118 (US); O'CONNELL, Dylan, Seattle, Washington 98121 (US); GOODMAN, Drew, Seattle, Washington 98121 (US); MERRILL, Lacey, Seattle, Washington 98104 (US); CHANDRASEKAR, Jagadeeswaran, Seattle, Washington 98121 (US)
(74) Representative: Grünecker Patent- und Rechtsanwälte PartG mbB
(86) International application number: PCT/US2018/066915
(87) International publication number: WO 2019/135934

(56) References cited:
- EP-A1- 0 487 218
- WO-A1-2017/087281
- JP-A- 2001 269 196
- US-A1- 2010 209 975
- US-A1- 2016 145 292
- US-A1- 2016 282 357
- PASSADORE M ET AL: "Differential effects of distamycin analogues on amplification of human gene sequences by polymerase-chain reaction", BIOCHEMICAL JOURNAL, vol. 308, no. 2, 1 June 1995 (1995-06-01), GB, pages 513 - 519, XP055828358, ISSN: 0264-6021, Retrieved from the Internet <URL:https://www.ncbi.nlm.nih.gov/pmc/articles/PMC1136955/pdf/biochemj00062-0153.pdf> [retrieved on 20210721], DOI: 10.1042/bj3080513
- HAHN ET AL: "Distamycins and netropsin as inhibitors of RNA and DNA polymerases", PHARMACOLOGY & THERAPEUTICS. PART A: CHEMOTHERAPY, TOXICOLOGY AND METABOLIC INHIBITORS, ELSEVIER SCIENCE, vol. 1, no. 4, 1 January 1977 (1977-01-01), pages 475 - 485, XP023855145, ISSN: 0362-5478, [retrieved on 19770101], DOI: 10.1016/0362-5478(77)90007-9
- MOTE JR. ET AL.: "A DNA Minor Groove-binding Ligand Both Potentiates and Arrests Transcription by RNA Polymerase II: Elongation Factor SII Enables Readthrough at Arrest Sites", J MOL BIOL, vol. 236, no. 3, 25 February 1994 (1994-02-25), pages 725 - 737, XP024008502, DOI: doi:10.1006/jmbi.1994.1185

## Description

### STATEMENT REGARDING SEQUENCE LISTING

The Sequence Listing associated with this application is provided in text format in lieu of a paper copy, and is hereby incorporated by reference into the specification. The name of the text file containing the Sequence Listing is 870225_422WO_SEQUENCE_LISTING.txt. The text file is 3.3 KB, was created on December 19, 2018, and is being submitted electronically via EFS-Web.

### TECHNICAL FIELD

The present invention relates generally to methods and compositions for the polymerization of nucleic acids and/or influencing enzyme performance.

### BACKGROUND

Measurement of biomolecules is a foundation of modern medicine and is broadly used in medical research, and more specifically in diagnostics and therapy, as well in drug development. Nucleic acids encode the necessary information for living things to function and reproduce, and are essentially a blueprint for life. Determining such blueprints is useful in pure research as well as in applied sciences. In medicine, sequencing can be used for diagnosis and to develop treatments for a variety of pathologies, including cancer, heart disease, autoimmune disorders, multiple sclerosis, and obesity. In industry, sequencing can be used to design improved enzymatic processes or synthetic organisms. In biology, this tool can be used to study the health of ecosystems, for example, and thus have a broad range of utility. Similarly, measurement of proteins and other biomolecules has provided markers and understanding of disease and pathogenic propagation.

An individual's unique DNA sequence provides valuable information concerning their susceptibility to certain diseases. It also provides patients with the opportunity to screen for early detection and/or to receive preventative treatment. Furthermore, given a patient's individual blueprint, clinicians will be able to administer personalized therapy to maximize drug efficacy and/or to minimize the risk of an adverse drug response. Similarly, determining the blueprint of pathogenic organisms can lead to new treatments for infectious diseases and more robust pathogen surveillance. Low cost, whole genome DNA sequencing will provide the foundation for modern medicine. To achieve this goal, sequencing technologies must continue to advance with respect to throughput, accuracy, and read length.

Over the last decade, a multitude of next generation DNA sequencing technologies have become commercially available and have dramatically reduced the cost of sequencing whole genomes. These include sequencing by synthesis ("SBS") platforms (Illumina, Inc., 454 Life Sciences, Ion Torrent, Pacific Biosciences) and analogous ligation based platforms (Complete Genomics, Life Technologies Corporation). A number of other technologies are being developed that utilize a wide variety of sample processing and detection methods. For example, GnuBio, Inc. (Cambridge, Mass.) uses picoliter reaction vessels to control millions of discreet probe sequencing reactions, whereas Halcyon Molecular (Redwood City, Calif.) was attempting to develop technology for direct DNA measurement using a transmission electron microscope.

Nanopore based nucleic acid sequencing is a compelling approach that has been widely studied. Kasianowicz et al. (Proc. Natl. Acad. Sci. USA 93: 13770-13773, 1996) characterized single-stranded polynucleotides as they were electrically translocated through an alpha hemolysin nanopore embedded in a lipid bilayer. It was demonstrated that during polynucleotide translocation partial blockage of the nanopore aperture could be measured as a decrease in ionic current. Polynucleotide sequencing in nanopores, however, is burdened by having to resolve tightly spaced bases (0.34 nm) with small signal differences immersed in significant background noise. The measurement challenge of single base resolution in a nanopore is made more demanding due to the rapid translocation rates observed for polynucleotides, which are typically on the order of 1 base per microsecond. Translocation speed can be reduced by adjusting run parameters such as voltage, salt composition, pH, temperature, and viscosity, to name a few. However, such adjustments have been unable to reduce translocation speed to a level that allows for single base resolution.

Stratos Genomics has developed a method called Sequencing by Expansion ("SBX") that uses a biochemical process to transcribe the sequence of DNA onto a measurable polymer called an "Xpandomer" (Kokoris et al., U.S. Pat. No. 7,939,259, "High Throughput Nucleic Acid Sequencing by Expansion"). The transcribed sequence is encoded along the Xpandomer backbone in high signal-to-noise reporters that are separated by ~10 nm and are designed for high-signal-to-noise, well-differentiated responses. These differences provide significant performance enhancements in sequence read efficiency and accuracy of Xpandomers relative to native DNA. Xpandomers can enable several next generation DNA sequencing detection technologies and are well suited to nanopore sequencing.

Xpandomers are generated from non-natural nucleotide analogs, termed XNTPs (see US published patent application no. US20160145292A1 to Kokoris et al., characterized by lengthy substituents that enable the Xpandomer backbone to be expanded following synthesis. Because of their atypical structures, XNTPs, as well as other nucleotide analogs (e.g., nucleotide analogs modified with detectable label moieties) introduce novel challenges as substrates for currently available DNA polymerases. Published PCT application no. WO2017/087281 to Kokoris et al., describes engineered DP04 polymerase variants with enhanced primer extension activity utilizing non-natural, bulky nucleotide analogues as substrates.

Within the DNA template itself, certain nucleotide sequence motifs are known to present additional replication challenges to DNA polymerases. Of particular consequence are runs of homopolymers, or short repeated DNA sequences, which can trigger slipped-strand mispairing, or "replication slippage". Replication slippage is thought to encompass the following steps: (i) copying of the first repeat by the replication machinery, (ii) replication pausing and dissociation of the polymerase from the newly synthesized end, (iii) unpairing of the newly synthesized strand and its pairing with the second repeat, and (iv) resumption of DNA synthesis. Arrest of the replication machinery within a repeated region thus results in misalignment of primer and template. In vivo, misalignment of two DNA strands during replication can lead to DNA rearrangements such as deletions or duplications of varying lengths. In vitro, replication slippage results in replication errors at the site of the slippage event. Such reduction in polymerase processivity, or accuracy, significantly impairs the particular application or desired genetic manipulation.

JP 2001/269196 discloses a method for quantitatively determining a nucleic acid in a test object such as a small amount of a gene or an mRNA in a specimen. EP 0487218A1 relates to a method of detecting or quantifying a target nucleic acid in a sample including reacting the amplified region with a fluorescent pigment, the fluorescent property of which is changed upon reaction to or exposure with nucleic acid.

Thus, new methods and compositions for enhancing polymerase reactions under conditions including one or more reagents with atypical structures are necessary, e.g., in sequencing by expansion (SBX) and other applications in biotechnology and biomedicine (DNA amplification, conventional sequencing, labeling, detection, cloning, etc.), and would find value in the art. The present invention fulfills these needs and provides further related advantages.

All of the subject matter discussed in the Background section is not necessarily prior art and should not be assumed to be prior art merely as a result of its discussion in the Background section. Along these lines, any recognition of problems in the prior art discussed in the Background section or associated with such subject matter should not be treated as prior art unless expressly stated to be prior art. Instead, the discussion of any subject matter in the Background section should be treated as part of the inventor's approach to the particular problem, which in and of itself may also be inventive.

### SUMMARY

In brief, the present disclosure relates to methods and compositions that enhance nucleic acid polymerase activity. In certain embodiments polymerase activity is enhanced in polymerization reactions under conditions that introduce one or more challenges to the polymerase, e.g., conditions that include non-natural nucleotide analog substrates or template motifs that impair polymerase processivity. Such enhancement is achieved by supplementing a polymerization reaction with one or more additives from a class of compounds known in the art as "minor groove binders" (MGB). Surprisingly and advantageously, the inventors have found that certain MGBs enhance polymerase activity significantly, particularly with non-natural, highly substituted nucleotide analog substrates.

The present invention is defined by the appended claims.

In one aspect, the invention provides a method of enhancing a nucleic acid polymerase reaction, the method including the steps of forming a nucleic acid polymerase reaction composition including a template nucleic acid, a nucleic acid polymerase, a mixture of nucleotides or nucleotide analogs, at least one minor groove binding moiety; and incubating the nucleic acid polymerase reaction composition under conditions allowing a nucleic acid polymerization reaction, wherein the at least one minor groove binding moiety increases the processivity, rate, or fidelity of the nucleic acid polymerase reaction, as further specified in the appended claims. In one embodiment, the at least one minor groove binding moiety increases the length of a resulting nucleic acid product compared to a nucleic acid polymerase reaction lacking the minor groove binding moiety. In another embodiment, the at least one minor groove binding moiety is selected from the group consisting of distamycin A and synthetic analogs thereof, netropsin, (+)-CC-1065, duocarmycins, pyrrolobenzodiazepines, trabectin and analogs thereof, Hoechst dyes and derivatives thereof, lexitropsin, thiazotropsin A, diamidines, and polyamides. In certain embodiments, the at least one minor groove binding moiety is a Hoechst dye. In other embodiments, at least one minor groove binding moiety includes a plurality of minor groove binding moieties. In some embodiments, the plurality of minor groove binding moieties includes minor groove binding moieties of different structural classes. In other embodiments, the nucleic acid polymerase is a DNA polymerase. In certain embodiments, the DNA polymerase is DPO4 or a variant thereof. In other embodiments, the mixture of nucleotides or nucleotide analogs is a mixture of nucleotide analogs comprising nucleoside triphosphoramidates, wherein each of the nucleoside triphosphoramidates comprises a nucleobase selected from the group consisting of adenine, guanine, thymine, and cytosine and a polymeric tether moiety, wherein a first end of the polymeric tether moiety is attached to the nucleobase and a second end of the polymeric tether moiety is attached to the alpha phosphate of the nucleoside triphosphoramidate to provide for expansion of the nucleotide analogs by cleavage of the phosphoramidate bond. In some embodiments, the nucleic acid polymerization reaction produces an expandable polymer of nucleotide analogs, wherein the expandable polymer encodes the nucleobase sequence information of the template nucleic acid. In other embodiments, the conditions for allowing a nucleic acid polymerization reaction comprise a suitable polymerization buffer and an oligonucleotide primer. In further embodiments, the suitable buffer comprises Tris OAc, NH₄OAc, PEG, DMF, polyphosphate 60, and MnClz. In other embodiments, the reaction mixture further includes a nucleic acid intercalating agent. In other embodiments, the reaction mixture further includes a polyanion recognition moiety. In further embodiments, the mixture of nucleotides or nucleotide analogs includes nucleotide analogs comprising a detectable label. In yet other embodiments, the detectable label is an optically detectable label selected from the group consisting of luminescent, chemiluminescent, fluorescent, fluorogenic, chromophoric or chromogenic labels.

In another aspect, the invention provides a composition including at least one minor groove binding moiety and a mixture of nucleotide analogs wherein the at least one minor groove binding moiety increases the number and accuracy of nucleotide analogs incorporated into a daughter strand during a template-dependent polymerization reaction relative to an identical polymerization reaction absent the at least one minor groove binding moiety, as further specified in the appended claims. In one embodiment, the at least one minor groove binding moiety is selected from the group consisting of distamycin A and synthetic analogs thereof, netropsin, (+)-CC-1065, duocarmycins, pyrrolobenzodiazepines, trabectin and analogs thereof, Hoechst dyes and derivatives thereof, lexitropsin, thiazotropsin A, diamidines, and polyamides. In certain embodiments, the at least one minor groove binding moiety is a Hoechst dye. In other embodiments, the at least one minor groove binding moiety comprises a plurality of minor groove binding moieties. In some embodiments, the plurality of minor groove binding moieties comprises minor groove binding moieties of different structural classes. In some embodiments, the mixture of nucleotide analogs comprises nucleoside triphosphoramidates, wherein each of the nucleoside triphosphoramidates comprises a nucleobase selected from the group consisting of adenine, guanine, thymine, and cytosine and a polymeric tether moiety, wherein a first end of the polymeric tether moiety is attached to the nucleobase and a second end of the polymeric ether moiety is attached to the alpha phosphate of the nucleoside triphosphoramidate to provide for expansion of the nucleotide analogs by cleavage of the phosphoramidate bond. In other embodiments, the composition further includes a buffer including Tris OAc, NH₄OAc, PEG, DMF, polyphosphate 60, and MnClz. In other embodiments, the composition further includes a DNA intercalating agent. In other embodiments, the composition further includes a polyanion recognition moiety. In certain embodiments, the mixture of nucleotide analogs includes nucleotide analogs including a detectable label. In some embodiments, the detectable label is an optically detectable label selected from the group consisting of luminescent, chemiluminescent, fluorescent, fluorogenic, chromophoric or chromogenic labels.

In another aspect, the invention provides a method of sequencing a DNA template, the method including the steps of forming a DNA polymerase reaction composition including the DNA template, a replication primer that complexes with the template, a DNA polymerase, a mixture of nucleotides or nucleotide analogs, and at least one minor groove binding moiety, incubating the DNA polymerase reaction composition under conditions allowing a DNA polymerization reaction, wherein the at least one minor groove binding moiety increases the rate, fidelity or processivity of the DNA polymerase reaction; and determining the sequence of the nucleotides or nucleotide analogs in the resulting polymer of nucleotides or nucleotide analogs, as further specified in the appended claims. In some embodiments, wherein the at least one minor groove binding moiety is selected from the group consisting of distamycin A and synthetic analogs thereof, netropsin, (+)-CC-1065, duocarmycins, pyrrolobenzodiazepines, trabectin and analogs thereof, Hoechst dyes and derivatives thereof, lexitropsin, thiazotropsin A, diamidines, and polyamides. In other embodiments, the mixture of nucleotide analogs comprises nucleoside triphosphoramidates, wherein each of the nucleoside triphosphoramidates comprises a nucleobase selected from the group consisting of adenine, guanine, thymine, and cytosine and a polymeric tether moiety, wherein a first end of the polymeric tether moiety is attached to the nucleobase and a second end of the polymeric ether moiety is attached to the alpha phosphate of the nucleoside triphosphoramidate to provide for expansion of the nucleotide analogs by cleavage of the phosphoramidate bond. In other embodiments, the DNA polymerase is DPO4 or a variant thereof. In other embodiments, the resulting polymer of nucleotide analogs is an expandable polymer. In other embodiments, the method further includes the step of contacting the expandable polymer with a phosphoramidate cleavage agent to produce an expanded polymer of nucleotide analogs. In certain embodiments, the polymeric tether moiety of each of the nucleotide analogs comprises a reporter moiety unique to the nucleobase of the analog. In other embodiments, the reporter moieties produce a characteristic electronic signal. In yet other embodiments, the step of determining the sequence of the nucleotide analogs includes the step of translocating the expanded polymer of nucleotide analogs through a nanopore.

### BRIEF DESCRIPTION OF THE DRAWINGS

Exemplary features of the present disclosure, its nature and various advantages will be apparent from the accompanying drawings and the following detailed description of various embodiments. Non-limiting and non-exhaustive embodiments are described with reference to the accompanying drawings, wherein like labels or reference numbers refer to like parts throughout the various views unless otherwise specified. The sizes and relative positions of elements in the drawings are not necessarily drawn to scale. For example, the shapes of various elements are selected, enlarged, and positioned to improve drawing legibility. The particular shapes of the elements as drawn have been selected for ease of recognition in the drawings.
**FIGS. 1A****,** **1B****,** **1C****, and** **1D** are condensed schematics illustrating the main features of a generalized XNTP and their use in Sequencing by Expansion (SBX).
**FIG. 2** is a schematic illustrating more details of one embodiment of an XNTP.
**FIG. 3** is a schematic illustrating one embodiment of an Xpandomer passing through a biological nanopore.
**FIG. 4** is a gel showing primer extension products.
**FIG. 5** is a gel showing primer extension products.
**FIG. 6** is a gel showing primer extension products.
**FIGS. 7A and 7B** are histogram displays of populations of aligned reads of nanopore-derived sequences.

### DETAILED DESCRIPTION

In the following description, certain specific details are set forth in order to provide a thorough understanding of various embodiments. However, one skilled in the art will understand that the invention may be practiced without these details. In other instances, well-known structures have not been shown or described in detail to avoid unnecessarily obscuring descriptions of the embodiments. Unless the context requires otherwise, throughout the specification and claims which follow, the word "comprise" and synonyms and variations thereof, such as, "have", "include", "comprises" and "comprising" are to be construed in an open, inclusive sense, that is, as "including, but not limited to." The term "consisting essentially of" limits the scope of a claim to the specified materials or steps, or to those that do not materially affect the basic and novel characteristics of the claimed invention. Further, headings provided herein are for convenience only and do not interpret the scope or meaning of the claimed invention.

Reference throughout this specification to "one embodiment" or "an embodiment" and variations thereof means that a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments. Also, as used in this specification and the appended claims, the singular forms "a," "an," and "the" include plural referents unless the content clearly dictates otherwise.

It should also be noted that the conjunctive terms, "and" and "or" are generally employed in the broadest sense to include "and/or" unless the content and context clearly dictates inclusivity or exclusivity as the case may be. Thus, the use of the alternative (e.g., "or") should be understood to mean either one, both, or any combination thereof of the alternatives. In addition, the composition of "and" and "or" when recited herein as "and/or" is intended to encompass an embodiment that includes all of the associated items or ideas and one or more other alternative embodiments that include fewer than all of the associated items or ideas.

Where a range of values is provided herein, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either or both of those included limits are also included in the invention.

For example, any concentration range, percentage range, ratio range, or integer range provided herein is to be understood to include the value of any integer within the recited range and, when appropriate, fractions thereof (such as one tenth and one hundredth of an integer), unless otherwise indicated. Also, any number range recited herein relating to any physical feature, such as polymer subunits, size or thickness, are to be understood to include any integer within the recited range, unless otherwise indicated. As used herein, the term "about" means ± 20% of the indicated range, value, or structure, unless otherwise indicated.

It is to be understood that the terminology used herein is for the purpose of describing specific embodiments only and is not intended to be limiting. It is further to be understood that unless specifically defined herein, the terminology used herein is to be given its traditional meaning as known in the relevant art.

Any headings used within this document are only being utilized to expedite its review by the reader.

### Definitions

As used herein, "nucleic acids", also called polynucleotides, are covalently linked series of nucleotides in which the 3' position of the pentose of one nucleotide is joined by a phosphodiester group to the 5' position of the next. A nucleic acid molecule can be deoxyribonucleic acid (DNA), ribonucleic acid (RNA), or a combination of both. DNA (deoxyribonucleic acid) and RNA (ribonucleic acid) are biologically occurring polynucleotides in which the nucleotide residues are linked in a specific sequence by phosphodiester linkages. As used herein, the terms "nucleic acid", "polynucleotide" or "oligonucleotide" encompass any polymer compound having a linear backbone of nucleotides. Oligonucleotides, also termed oligomers, are generally shorter chained polynucleotides. Nucleic acids are generally referred to as "target nucleic acids" or "target sequence" if targeted for sequencing.

As used herein, "polymerization" refers to an in vitro method for making a new strand of nucleic acid or elongating an existing nucleic acid (i.e., DNA or RNA) in a template dependent manner. Polymerization, according to the invention, includes primer extension, which replicates the sequence of a polynucleotide template sequence with the use of a polymerase. Nucleic acid polymerization (e.g., primer extension) results in the incorporation of nucleotides or nucleotide analogs into a polynucleotide (i.e., a primer), thereby forming a new nucleic acid molecule complementary to the polynucleotide template. The formed nucleic acid molecule can be used for single molecule sequencing or as templates to synthesize additional nucleic acid molecules.

As used herein, the term "template dependent manner" is intended to refer to a process that involves the template dependent extension of a primer molecule (e.g., DNA synthesis by DNA polymerase). The term "template dependent manner" refers to polynucleotide synthesis of RNA or DNA wherein the sequence of the newly synthesized strand of polynucleotide is dictated by the well-known rules of complementary base pairing (see, for example, Watson, J. D. et al., In: Molecular Biology of the Gene, 4th Ed., W. A. Benjamin, Inc., Menlo Park, Calif. (1987)).

As used herein, "nucleic acid polymerase" is an enzyme generally for joining 3'-OH 5'-triphosphate nucleotides, oligomers, and their analogs. Polymerases include, but are not limited to, DNA-dependent DNA polymerases, DNA-dependent RNA polymerases, RNA-dependent DNA polymerases, RNA-dependent RNA polymerases, T7 DNA polymerase, T3 DNA polymerase, T4 DNA polymerase, T7 RNA polymerase, T3 RNA polymerase, SP6 RNA polymerase, DNA polymerase 1, Klenow fragment, *Thermophilus aquaticus* DNA polymerase, Tth DNA polymerase, VentR^{®} DNA polymerase (New England Biolabs), Deep VentR^{®} DNA polymerase (New England Biolabs), Bst DNA Polymerase Large Fragment, Stoeffel Fragment, 9° N DNA Polymerase, 9° N DNA polymerase, Pfu DNA Polymerase, Tfl DNA Polymerase, Tth DNA Polymerase, RepliPHI Phi29 Polymerase, Tli DNA polymerase, eukaryotic DNA polymerase beta, telomerase, Therminator^{™} polymerase (New England Biolabs), KOD HiFi^{™} DNA polymerase (Novagen), KOD1 DNA polymerase, Q-beta replicase, terminal transferase, AMV reverse transcriptase, M-MLV reverse transcriptase, Phi6 reverse transcriptase, HIV-1 reverse transcriptase. A polymerase according to the invention can be a variant, mutant, or chimeric polymerase.

As used herein, a "DPO4-type DNA polymerase" is a DNA polymerase naturally expressed by the archaea, Sulfolobus solfataricus, or a related Y-family DNA polymerase, which generally function in the replication of damaged DNA by a process known as translesion synthesis (TLS). Y-family DNA polymerases are homologous to the DPO4 polymerase (e.g., as listed in SEQ ID NO:1); examples include the prokaryotic enzymes, Polll, PollV, PolV, the archaeal enzyme, Dbh, and the eukaryotic enzymes, Rev3p, Revlp, Pol η, REV3, REV1, Pol 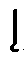 , and Pol κ DNA polymerases, as well as chimeras thereof. A modified recombinant DPO4-type DNA polymerase includes one or more mutations relative to naturally-occurring wild-type DPO4-type DNA polymerases, for example, one or more mutations that increase the ability to utilize bulky nucleotide analogs as substrates or another polymerase property, and may include additional alterations or modifications over the wild-type DPO4-type DNA polymerase, such as one or more deletions, insertions, and/or fusions of additional peptide or protein sequences (e.g., for immobilizing the polymerase on a surface or otherwise tagging the polymerase enzyme). Examples of variant polymerase according to the invention are the variants of Sulfolobus sulfataricus DPO4 described in published PCT patent application WO2017/087281 A1.

The polymerase activity of any of the above enzymes can be determined by means known in the art. For example, the nucleotide incorporation assay of Hogrefe et al. (Methods in Enzymol. Vol. 334, pp. 91-116 (2001)) can be used to measure the rate of polymerization. Briefly, polymerase activity can be measured as the rate of incorporation of ³²P-dCTP into activated salmon sperm DNA (purchased from Pharmacia; for activation protocol see C. C. Richardson, Procedures in Nucl. Acid Res. (Cantoni and Davies, eds.), p. 263-276 (1966) at p. 264). The reaction buffer can be, for example, 50 mM Tris-HCl (pH 8.0), 5 mM MgClz, 1 mM dithiothreitol (DTT), 50 µg/ml bovine serum albumin (BSA), and 4% (v/v) glycerol. Nucleotide substrates and DNA are used in large excess, typically at least 10 times the Km for the polymerase being assayed, e.g., 200 µgM each of dATP, dTTP, and dGTP, 195 µgM of dCTP plus 5 µg M of labeled dCTP, and 250 µg/ml of activated DNA. The reactions are quenched on ice, and aliquots of the reaction mixture are spotted onto ion exchange filters (e.g., Whatman DE81). Unincorporated nucleotide is washed through, followed by scintillation counting to measure incorporated radioactivity. As used herein, "enhanced rate" refers to an increase of 5-10%, 10-50%, or 50-100% or more, as compared to a polymerization reaction that lacks an additive that increases rate as defined herein.

As used herein, "processivity" refers to the extent of polymerization by a nucleic acid polymerase during a single contact between the polymerase and its template, i.e, its property to continue to act on a substrate instead of dissociating therefrom. The extent of polymerization refers to the number of nucleotides or nucleotide analogs added by the polymerase during a single contact between the polymerase and its template. Processivity can depend on the nature of the polymerase, the sequence of a template, the structure of the nucleotide or nucleotide analog substrates, and the reaction conditions, for example, salt concentration, temperature or the presence of specific additives.

As used herein, "enhanced processivity" refers to an increase of 5-10%, 10-50%, or 50-100% or more, as compared to a polymerization reaction that lacks an additive that increases processivity as defined herein. Methods for measuring processivity of a nucleic acid polymerase are generally known in the art, e.g., as described in Sambrook et al. 1989, In Molecular Cloning, 2nd Edition, CSH Press, 7.79-7.83 and 13.8, as described in U.S. published patent application no. 2002/0119467, published PCT application no. WO01/92501 and in U.S. Pat. No. 5,972,603.

The term "fidelity" as used herein refers to the accuracy of nucleic acid polymerization by template-dependent nucleic acid polymerase. The fidelity of a DNA polymerase is measured by the error rate (the frequency of incorporating an inaccurate nucleotide, i.e., a nucleotide that is not incorporated at a template-dependent manner). The fidelity or error rate of a DNA polymerase may be measured using assays known to the art (see for example, Lundburg et al., 1991 Gene, 108:1-6). As used herein, "enhanced fidelity" refers to an increase of 5-10%, 10-50%, or 50-100% or more, as compared to a polymerization reaction that lacks an additive that increases fidelity as defined herein.

"Primer extension reaction" means a reaction between a target-primer hybrid and a nucleotide or nucleotide analog which results in the addition of the nucleotide or nucleotide analog to a 3'-end of the primer such that the incorporated nucleotide or nucleotide analog is complementary to the corresponding nucleotide of the target polynucleotide. Primer extension reagents typically include (i) a polymerase enzyme; (ii) a buffer; and (iii) one or more extendible nucleotides or nucleotide analogs. Primer extension reactions can be used to measure the length of a resulting nucleic acid product under particular experimental conditions and to determine the effect of various polymerase reaction additives on polymerase activity by comparing the lengths of the extended primer products by, e.g., gel electrophoresis.

"Minor groove binding moieties" (referred to also as "MGBs") as used herein is a term well-accepted in the art to refer to a diverse group of small molecule DNA-binding agents whose putative mechanism of action is with the minor groove of double-stranded DNA. In general, the structure of the MGBs adopt a curved conformation that complements the curve of the minor groove of B-type DNA. However, it is to be understood that, according to the present invention, the manner in which the MGBs interact with a template nucleic acid during a polymerase reaction has not been determined and it is unclear whether mechanism of action based on minor groove binding is material to the invention. The inventors use this term solely due to its long-standing use in the art and not to describe any limiting mechanism of action. MGBs of the invention may be derived from natural products or synthetic. MGBs may be any analog or derivative of any known MGBs. Exemplary MGBs include, but are not limited to, Hoechst dyes and derivatives (e.g., Hoechst 33258, Hoechst 34580, Hoechst 33342), netropsin, distamycin A and synthetic analogs thereof (e.g., lexitropsin and thiazotropsin A), (+)-CC-1065, duocarmycins, pyrrolobenzodiazepines, trabectin and analogs, diamidines (e.g., DAPI, berenil, pentamidine, DB293, and 3,6-diaminoacridine hydrochloride), and polyamides.

The term "plurality" as used herein refers to "at least two."

"XNTP" is an expandable, 5' triphosphate modified nucleotide substrate compatible with template dependent enzymatic polymerization. An XNTP has two distinct functional components; namely, a nucleobase 5'-triphosphoramidate and a tether that is attached within each nucleoside triphosphoramidate at positions that allow for controlled expansion by intra-nucleotide cleavage of the phosphoramidate bond. XNTPs are exemplary "non-natural, highly substituted nucleotide analog substrates", as used herein. Exemplary XNTPs and methods of making the same are described, e.g., in Applicants' published PCT application no. WO2016/081871.

"Xpandomer intermediate" is an intermediate product (also referred to herein as a "daughter strand") assembled from XNTPs, and is formed by polymerase-mediated template-directed assembly of XNTPs using a target nucleic acid template. The newly synthesized Xpandomer intermediate is a constrained Xpandomer. Under a process step in which the phosphoramidate bonds provided by the XNTPs are cleaved, the constrained Xpandomer is no longer constrained and is the Xpandomer product which is extended as the tethers are stretched out.

"Xpandomer" or "Xpandomer product" is a synthetic molecular construct produced by expansion of a constrained Xpandomer, which is itself synthesized by template-directed assembly of XNTP substrates. The Xpandomer is elongated relative to the target template it was produced from. It is composed of a concatenation of subunits, each subunit a motif, each motif a member of a library, comprising sequence information, a tether and optionally, a portion, or all of the substrate, all of which are derived from the formative substrate construct. The Xpandomer is designed to expand to be longer than the target template thereby lowering the linear density of the sequence information of the target template along its length. In addition, the Xpandomer optionally provides a platform for increasing the size and abundance of reporters which in turn improves signal to noise for detection. Lower linear information density and stronger signals increase the resolution and reduce sensitivity requirements to detect and decode the sequence of the template strand.

"Tether" or "tether member" refers to a polymer or molecular construct having a generally linear dimension and with an end moiety at each of two opposing ends. A tether is attached to a nucleoside triphosphoramidate with a linkage at end moiety to form an XNTP. The linkages serve to constrain the tether in a "constrained configuration". Tethers have a "constrained configuration" and an "expanded configuration". The constrained configuration is found in XNTPs and in the daughter strand, or Xpandomer intermediate. The constrained configuration of the tether is the precursor to the expanded configuration, as found in Xpandomer products. The transition from the constrained configuration to the expanded configuration results cleaving of selectively cleavable phosphoramidate bonds. Tethers comprise one or more reporters or reporter constructs along its length that can encode sequence information of substrates. The tether provides a means to expand the length of the Xpandomer and thereby lower the sequence information linear density.

"Tether element" or "tether segment" is a polymer having a generally linear dimension with two terminal ends, where the ends form end-linkages for concatenating the tether elements. Tether elements are segments of tether. Such polymers can include, but are not limited to: polyethylene glycols, polyglycols, polypyridines, polyisocyanides, polyisocyanates, poly(triarylmethyl)methacrylates, polyaldehydes, polypyrrolinones, polyureas, polyglycol phosphodiesters, polyacrylates, polymethacrylates, polyacrylamides, polyvinyl esters, polystyrenes, polyamides, polyurethanes, polycarbonates, polybutyrates, polybutadienes, polybutyrolactones, polypyrrolidinones, polyvinylphosphonates, polyacetamides, polysaccharides, polyhyaluranates, polyamides, polyimides, polyesters, polyethylenes, polypropylenes, polystyrenes, polycarbonates, polyterephthalates, polysilanes, polyurethanes, polyethers, polyamino acids, polyglycines, polyprolines, N-substituted polylysine, polypeptides, side-chain N-substituted peptides, poly-N-substituted glycine, peptoids, side-chain carboxyl-substituted peptides, homopeptides, oligonucleotides, ribonucleic acid oligonucleotides, deoxynucleic acid oligonucleotides, oligonucleotides modified to prevent Watson-Crick base pairing, oligonucleotide analogs, polycytidylic acid, polyadenylic acid, polyuridylic acid, polythymidine, polyphosphate, polynucleotides, polyribonucleotides, polyethylene glycol-phosphodiesters, peptide polynucleotide analogues, threosyl-polynucleotide analogues, glycol-polynucleotide analogues, morpholino-polynucleotide analogues, locked nucleotide oligomer analogues, polypeptide analogues, branched polymers, comb polymers, star polymers, dendritic polymers, random, gradient and block copolymers, anionic polymers, cationic polymers, polymers forming stem-loops, rigid segments and flexible segments.

A "reporter" is composed of one or more reporter elements. Reporters serve to parse the genetic information of the target nucleic acid.

"Reporter construct" comprises one or more reporters that can produce a detectable signal(s), wherein the detectable signal(s) generally contain sequence information. This signal information is termed the "reporter code" and is subsequently decoded into genetic sequence data. A reporter construct may also comprise tether segments or other architectural components including polymers, graft copolymers, block copolymers, affinity ligands, oligomers, haptens, aptamers, dendrimers, linkage groups or affinity binding group (e.g., biotin).

"Reporter Code" is the genetic information from a measured signal of a reporter construct. The reporter code is decoded to provide sequence-specific genetic information data.

### Methods for enhanced nucleic acid polymerization with minor groove binding moieties (MGBs).

MGBs are a diverse group of small molecule DNA-binding agents whose putative mechanism of action is with the minor groove of double-stranded DNA (for a general review, see, e.g., J.M. Withers, G. Padroni, S.M. Pauff, A.W. Clark, S.P. Mackay and G.A. Burley, 5.07 - DNA Minor Groove Binders as Therapeutic Agents, In Comprehensive Supramolecular Chemistry II, edited by Jerry L. Atwood, Elsevier, Oxford, 2017, Pages 149-178, ISBN 9780128031995, https://doi.org/10.1016/B978-0-12-409547-2.12561-2). In general, the structure of the MGBs adopt a curved conformation that complements the curve of the minor groove of B-type DNA. MGBs may be any analog or derivative of any known MGBs. Exemplary MGBs include, but are not limited to, Hoechst dyes and derivatives (e.g., Hoechst 33258, Hoechst 34580, Hoechst 33342), netropsin, distamycin A and synthetic analogs thereof (e.g., lexitropsin and thiazotropsin A), (+)-CC-1065, duocarmycins, pyrrolobenzodiazepines, trabectin and analogs, diamidines (e.g., DAPI, berenil, pentamidine, DB293, and 3,6-diaminoacridine hydrochloride), and polyamides. MGBs are available from many commercial sources, such as MilliporeSigma Corporation (St. Louis, MO).

According to certain embodiments of the invention, MGBs may enhance any nucleic acid polymerization reaction or improve the properties of the resulting nucleic acid, e.g., the length or accuracy of the reaction product. Polymerization reactions include, e.g., primer extension reactions, PCR, mutagenesis, isothermal amplification, DNA sequencing, and probe labeling. Such methods are well known in the art. Enhancement may be provided by stimulating nucleotide incorporation through mechanisms such as increasing processivity of the polymerase (i.e. reducing dissociation of the polymerase from the template), increasing the rate of substrate binding or enzymatic catalysis, and increasing the accuracy or fidelity of nucleotide incorporation. In addition, enhancement may be provided by reducing impediments in the nucleic acid template, such as secondary structure and duplex DNA. Overcoming or improving such impediments through the addition of MGBs can allow polymerization reactions to occur more accurately or efficiently, or allow the use of lower denaturation/extension temperatures or isothermal temperatures.

In some embodiments, MGBs may be used in combination with other PEM additive classes to enhance a polymerase reaction.

### Sequencing by expansion (SBX)

One exemplary polymerase reaction that can be enhanced with MGBs is the polymerization of the non-natural nucleotide analogs known as "XNTPs", which forms the basis of the "Sequencing by Expansion" (SBX) protocol, developed by Stratos Genomics (see, e.g., Kokoris et al., U.S. Pat. No. 7,939,259, "High Throughput Nucleic Acid Sequencing by Expansion"). In general terms, SBX uses this biochemical polymerization to transcribe the sequence of a DNA template onto a measurable polymer called an "Xpandomer". The transcribed sequence is encoded along the Xpandomer backbone in high signal-to-noise reporters that are separated by ~10 nm and are designed for high-signal-to-noise, well-differentiated responses. These differences provide significant performance enhancements in sequence read efficiency and accuracy of Xpandomers relative to native DNA. A generalized overview of the SBX process is depicted in FIG.s **1A - 1D****.**

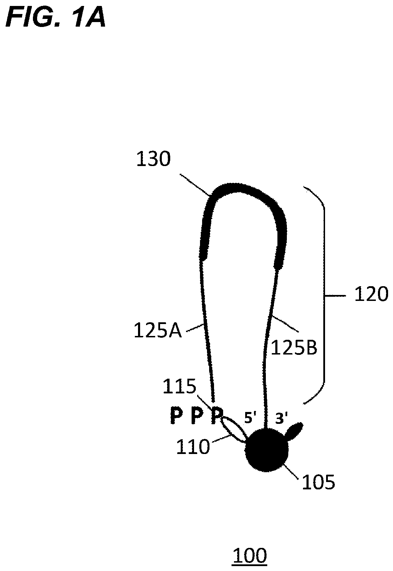

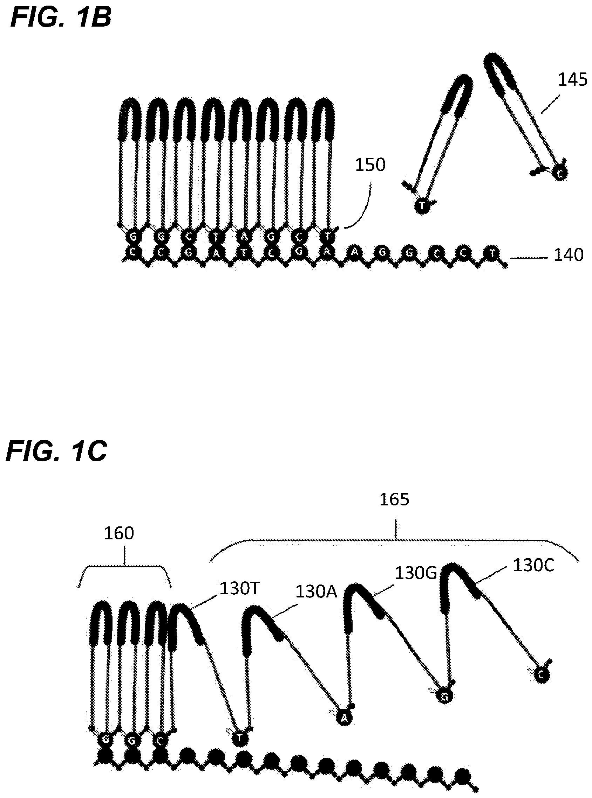

XNTPs are expandable, 5' triphosphate modified nucleotide substrates compatible with template dependent enzymatic polymerization. A highly simplified XNTP is illustrated in FIG. **1A**, which emphasizes the unique features of these nucleotide analogs: XNTP **100** has two distinct functional regions; namely, a selectively cleavable phosphoramidate bond **110**, linking the 5' α-phosphate **115** to the nucleobase **105**, and a tether **120** that is attached within the nucleoside triphosphoramidate at positions that allow for controlled expansion by intra-nucleotide cleavage of the phosphoramidate bond. The tether of the XNTP is comprised of linker arm moieties **125A** and **1258** separated by the selectively cleavable phosphoramidate bond. Each linker attaches to one end of a reporter **130** via a linking group (LG), as disclosed in U.S. Pat. No. 8,324,360 to Kokoris et al. XNTP **100** is illustrated in the "constrained configuration", characteristic of the XNTP substrates and the daughter strand following polymerization. The constrained configuration of polymerized XNTPs is the precursor to the expanded configuration, as found in Xpandomer products. The transition from the constrained configuration to the expanded configuration occurs upon scission of the P--N bond of the phosphoramidate within the primary backbone of the daughter strand.

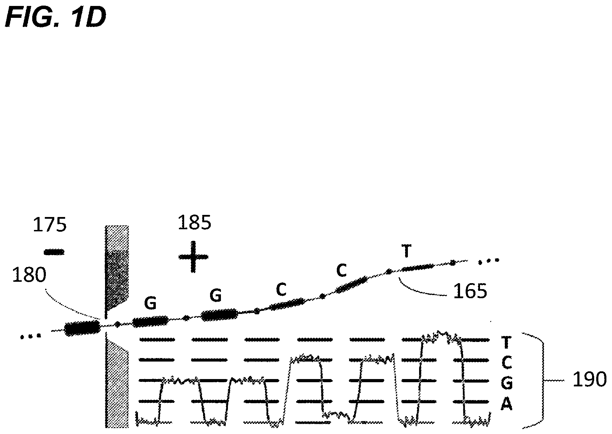

Synthesis of an Xpandomer is summarized in FIG.s **1B** and **1C**. During assembly, the monomeric XNTP substrates **145** (XATP, XCTP , XGTP, and XTTP), are polymerized on the extendable terminus of a nascent daughter strand **150** by a process of template-directed polymerization using single-stranded template **140** as a guide. Generally, this process is initiated from a primer and proceeds in the 5' to 3' direction. Generally, a DNA polymerase or other polymerase is used to form the daughter strand, and conditions are selected so that a complementary copy of the template strand is obtained. After the daughter strand is synthesized, the coupled tethers comprise the constrained Xpandomer that further comprises the daughter strand. Tethers in the daughter strand have the "constrained configuration" of the XNTP substrates. The constrained configuration of the tether is the precursor to the expanded configuration, as found the Xpandomer product. As shown in FIG. **1C**, the transition from the constrained configuration **160** to the expanded configuration **165** results from cleavage of the selectively cleavable phosphoramidate bonds (illustrated for simplicity by the unshaded ovals) within the primary backbone of the daughter strand. In this embodiment, the tethers comprise one or more reporters or reporter constructs, **130A, 130C, 130G,** or **130T,** specific for the nucleobase to which they are linked, thereby encoding the sequence information of the template. In this manner, the tethers provide a means to expand the length of the Xpandomer and lower the linear density of the sequence information of the parent strand. FIG. **1D** illustrates an Xpandomer **165** translocating through a nanopore **180,** from the cis reservoir **175** to the trans reservoir **185.** Upon passage through the nanopore, each of the reporters of the linearized Xpandomer (in this illustration, labeled "G", "C" and "T") generates a distinct and reproducible electronic signal (illustrated by superimposed trace **190),** specific for the nucleobase to which it is linked.

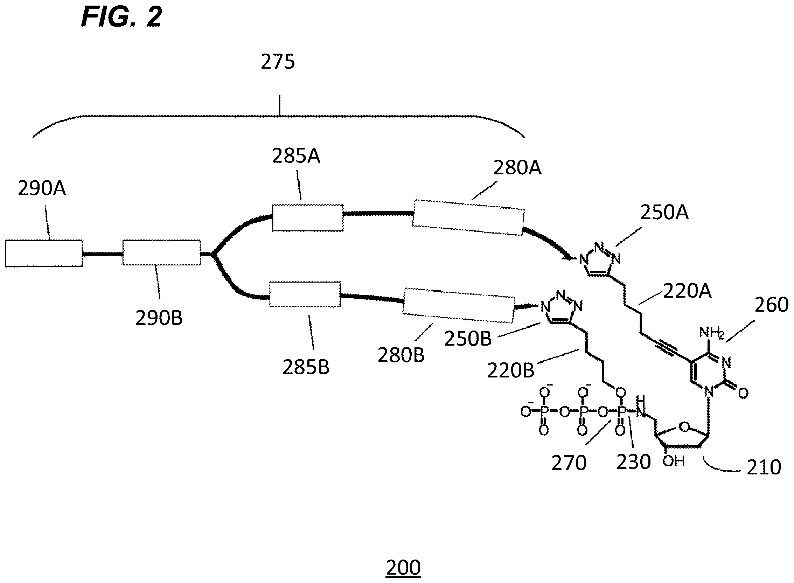

FIG. 2 depicts the generalized structure of an XNTP in more detail. XNTP **200** is comprised of nucleobase triphosphoramidate **210** with linker arm moieties **220A** and **220B** separated by selectively cleavable phosphoramidate bond **230.** Tethers are joined to the nucleoside triphosphoramidate at linking groups **250A** and **250B,** wherein a first tether end is joined to the heterocycle **260** (represented here by cytosine, though the heterocycle may be any one of the four standard nucleobases, A, C, G, or T) and the second tether end is joined to the alpha phosphate **270** of the nucleobase backbone. The skilled artisan will appreciate that many suitable coupling chemistries known in the art may be used to form the final XNTP substrate product, for example, tether conjugation may be accomplished through a triazole linkage.

In this embodiment, tether **275** is comprised of several functional elements, including enhancers **280A** and **280B,** reporter codes **285A** and **285B,** and translation control elements (TCEs) **290A** and **290B.** Each of these features performs a unique function during translocation of the Xpandomer through a nanopore and generation of a unique and reproducible electronic signal. Tether **275** is designed for translocation control by hybridization (TCH). As depicted, the TCEs provide a region of hybridization which can be duplexed to a complementary oligomer (CO) and are positioned adjacent to the reporter codes. Different reporter codes are sized to block ion flow through a nanopore at different measureable levels. Specific reporter codes can be efficiently synthesized using phosphoramidite chemistry typically used for oligonucleotide synthesis. Reporters can be designed by selecting a sequence of specific phosphoramidites from commercially available libraries. Such libraries include but are not limited to polyethylene glycol with lengths of 1 to 12 or more ethylene glycol units, aliphatic with lengths of 1 to 12 or more carbon units, deoxyadenosine (A), deoxycytosine (C), deoxyguanodine (G), deoxythymine (T), abasic (Q). The duplexed TCEs associated with the reporter codes also contribute to the ion current blockage, thus the combination of the reporter code and the TCE can be referred to as a "reporter". Following the reporter codes are the enhancers, which in one embodiment comprise spermine polymers.

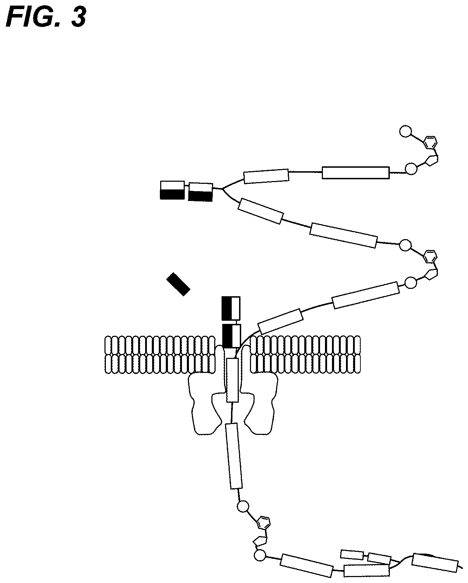

FIG. 3 shows one embodiment of a cleaved Xpandomer in the process of translocating an α-hemolysin nanopore. This biological nanopore is embedded into a lipid bilayer membrane which separates and electrically isolates two reservoirs of electrolytes. A typical electrolyte has 1 molar KCI buffered to a pH of 7.0. When a small voltage, typically 100 mV, is applied across the bilayer, the nanopore constricts the flow of ion current and is the primary resistance in the circuit. Xpandomer reporters are designed to give specific ion current blockage levels and sequence information can be read by measuring the sequence of ion current levels as the sequence of reporters translocate the nanopore.

The α-hemolysin nanopore is typically oriented so translocation occurs by entering the vestibule side and exiting the stem side. As shown in FIG. 3, the nanopore is oriented to capture the Xpandomer from the stem side first. This orientation is advantageous using the TCH method because it causes fewer blockage artifacts that occur when entering vestibule first. Unless indicated otherwise, stem side first will be the assumed translocation direction. As the Xpandomer translocates, a reporter enters the stem until its duplexed TCE stops at the stem entrance. The duplex is ~2.4 nm in diameter whereas the stem entrance is ~2.2 nm so the reporter is held in the stem until the complimentary strand 395 of the duplex disassociates (releases) whereupon translocation proceeds to the next reporter. The free complementary strand is highly disfavored from entering the nanopore because the Xpandomer is still translocating and diffuses away from the pore.

In one embodiment, each member of a reporter code (following the duplex) is formed by an ordered choice of phosphoramidites that can be selected from many commercial libraries. Each constituent phosphoramidite contributes to the net ion resistance according to its position in the nanopore (located after the duplex stop), its displacement, its charge, its interaction with the nanopore, its chemical and thermal environment and other factors. The charge on each phosphoramidite is due, in part, to the phosphate ion which has a nominal charge of -1 but is effectively reduced by counterion shielding. The force pulling on the duplex is due to these effective charges along the reporter which are acted upon by the local electric fields. Since each reporter can have a different charge distribution, it can exert a different force on the duplex for a given applied voltage. The force transmitted along the reporter backbone also serves to stretch the reporter out to give a repeatable blocking response.

### Compositions for enhanced nucleic acid polymerization with minor groove binding moieties (MGBs).

In one embodiment the present disclosure provides a composition comprising at least one MGB as disclosed herein and a plurality of nucleotides and/or nucleotide analogs. In another embodiment, the present disclosure provides a composition comprising at least one MGB as disclosed herein and a buffer. In another embodiment, the present disclosure provides a composition comprising at least one MGB as disclosed herein and a polynucleotide. In another embodiment, the present disclosure provides a composition comprising at least one MGB as disclosed herein and a protein, where optionally the protein is a polymerase including any of the polymerases described above.

In one embodiment, the present disclose provides an aqueous (water containing) composition comprising at least one MGB and a buffer, particularly a buffer suitable for conducting a DNA polymerization reaction, where Tris HCl is an exemplary buffer of this type. In one embodiment, the present disclosure provides a composition comprising at least one MGB and a DNA polymerase protein. In one embodiment, the present disclosure provides a composition comprising at least one MGB and a polynucleotide, e.g., a 20-90 mer, 20-60 mer, 30-90 mer, or a 30-60 mer, oligonucleotide. In one embodiment, the present disclosure provides a composition that comprises each of these components, i.e., an aqueous composition comprising at least one MGB, a buffer, a DNA polymerase protein and a polynucleotide.

In some embodiments, a MGB may be used in combination with another additive classes to enhance a polymerase reaction. One exemplary class of additives is polymerization enhancing moieties (PEMs). More information about the use of PEMs to enhance a polymerase reaction may be found in applicants' co-filed application titled ENHANCEMENT OF NUCLEIC ACID POLYMERIZATION BY AROMATIC COMPOUNDS.

### EXAMPLES

### Example 1

### Screen of Additives for Enhancement of XNTP Polymerization

The Sequencing by Expansion (SBX) methodology developed by the inventors provides significant performance enhancements in sequence read efficiency and accuracy of Xpandomers relative to native DNA. However, initial transcription of the sequence of the natural DNA template onto the measurable Xpandomer relies on the ability of DNA polymerase to utilize XNTPs as substrates (the generalized structure of an XNTP is discussed herein with reference to FIG. 1A and FIG. 2). The inventors have found that most DNA polymerases do not efficiently polymerize XNTPs. In an effort to improve the efficiency and accuracy of XNTP polymerization into Xpandomers, a wide variety of additives was screened for the ability to enhance DNA polymerase primer extension reactions using XNTPs as substrates.

A typical primer extension reaction includes the following reagents: 2 pmol primer, 2.2 pmol 45mer oligonucleotide template, 50 pmol of each XNTP (XATP, XCTP, XGTP, and XTTP), 50 mM Tris HCl, pH 6.79, 200 mM NaCl, 20% PEG, 5% NMS, 0.5 nmol polyphosphate 60.19, 0.3 mM MnCl₂, and 0.6 µg of purified recombinant DNA polymerase protein. Reactions are run for 1 hr at 23°C. Reaction products (i.e. constrained Xpandomers) are treated to cleave the phosphoramidate bonds, thereby generating linearized Xpandomers. Reaction products are analyzed using gel electrophoresis on 4-12% acrylamide gels to resolve and visualize Xpandomer products of different lengths. For the additive screen, reaction additives were typically tested in the micro to millimolar range.

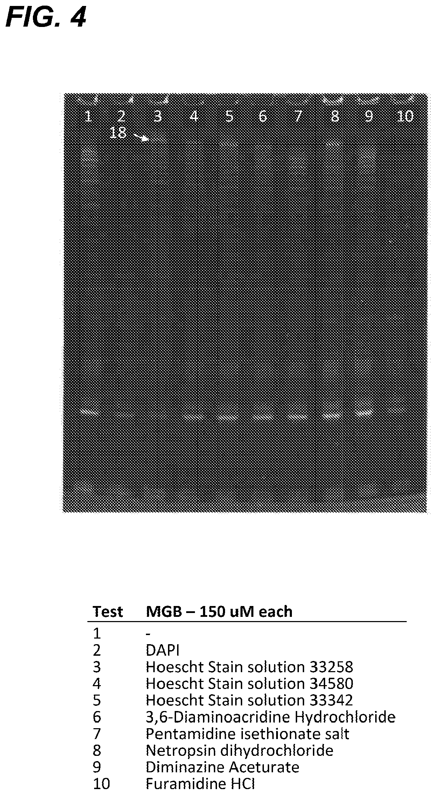

A summary of the additive screening results is set forth in Table 1 below. As expected based on prior experimentation, sodium polyphosphate and PEG8000 enhanced primer extension with XNTPs. However, the majority of the previously untested additives either had no effect or actually inhibited the primer extension reaction. Surprisingly, several known MGBs, including Hoescht 33258, Hoescht 34580, Hoescht 33342, 3,6-Diaminoacridine hydrochloride, and netropsin dihydrochloride significantly and reproducibly enhanced primer extension with XNTPs. A representative gel demonstrating this enhancement is presented in FIG. 4. As can be seen in lane 1, in the absence of additive, DNA polymerase extends the template bound primer with up to around 14 XNTPs under these conditions. The absence of the 14mer extension product in lanes 2 and 10 indicates that DAPI and furamidine have an inhibitory effect on the XNTP polymerization reaction. The extension products in lanes 3 through 9 appear to exceed the length of those in the lane 1 control (e.g., the arrow in lane 3 points to an 18mer product), demonstrating the enhancing effect of these MGBs on XNTP polymerization. Thus, MGBs became the focus for further optimization of XNTP polymerization.

**TABLE 1**

| **Additive** | **Extension Effect** |
|---|---|
| Sodium Polyphosphate | Necessary additive |
| Benzylamine | No effect |
| Glycerol | No effect |
| Dimethylformamide | No effect |
| NaCl | Beneficial |
| NiSO4 | Inhibitory |
| Triethylamine acetate | Inhibitory |
| Potassium Chloride | No effect |
| N-methyl succinimide | Beneficial |
| O-Phospho-L-tyrosine | Inhibitory |
| 2-Aminoterephthalic acid | Inhibitory |
| 5-Aminoisophthalic acid | Inhibitory |
| O-phospho-DL-serine | Inhibitory |
| Magnesium | Inhibitory |
| PEG6000 | Beneficial |
| PEG8000 | Necessary |
| PEG20000 | Beneficial |
| Ficoll 400 | No effect |
| Sphingosine | No effect |
| Arginine | Inhibitory |
| Betaine | Inhibitory |
| TMACI | No effect |
| Imidazole | No effect |
| SSB | Variable effects |
| BSA | Variable effects |
| Short oligonucleotides | Inhibitory |
| Tween 20 | No effect |
| Triton | No effect |
| Methyl triamine | No effect |
| Methyl amine diol | No effect |
| Piperazine diol | No efffect |
| C2 diamine diol | No effect |
| Tolyl diol | No effect |
| Sodium Hexanoate | No effect |
| N-Lauryl Sarcosine | Inhibitory |
| Dextran Sulfate sodium Salt | Inhibitory |
| Girards Reagent T | No effect |
| Cetylpyridinium chloride | Inhibitory |
| Dodecyltrimethylammonium chloride | Inhibitory |
| ASB-14 (amidosulfobetaine) | No effect |
| C7BzO | No effect |
| 3-(1-pyridinio)-1-propanesulfonate | No effect |
| TMAO | Variable effects |
| DMSO | Length increases |
| Propylene carbonate | Inhibitory |
| Acetonitrile | No effect |
| Tetrahydrofuran | No effect |
| Nitromethane | Inhibitory |
| Acetone | Beneficial |
| Piperdine | Inhibitory |
| Sodium docecyl sulfate | No effect |
| Urea | Beneficial |
| DAPI | No effect |
| Hoescht Stain solution 33258 | Beneficial |
| Hoescht Stain solution 34580 | Beneficial |
| Hoescht Stain solution 33342 | Beneficial |
| 3,6-Diaminoacridine Hydrochloride | Beneficial |
| Pentamidine isethionate salt | No effect |
| Netropsin dihydrochloride | Beneficial |
| Diminazine Aceturate | No effect |
| Furamidine HCl | Inhibitory |
| CDPI3 | Variable effects |

### Example 2

### Optimization of MGB-Mediated Enhancement of XNTP Polymerization

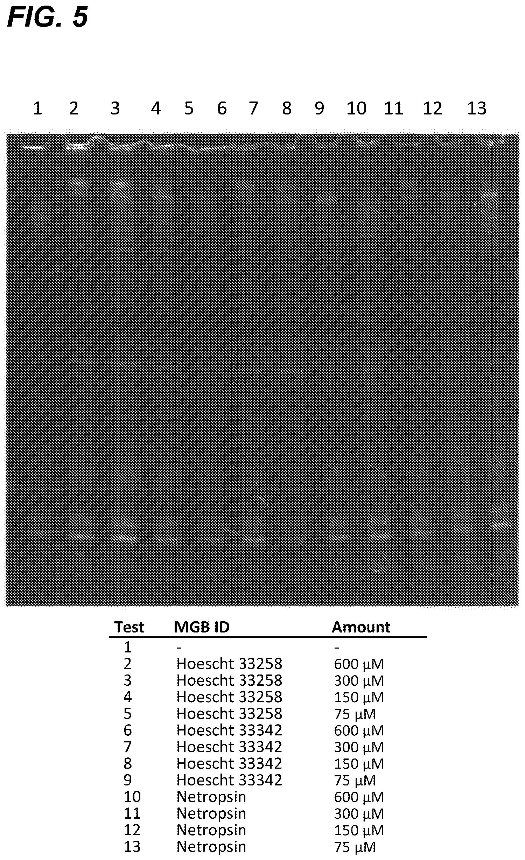

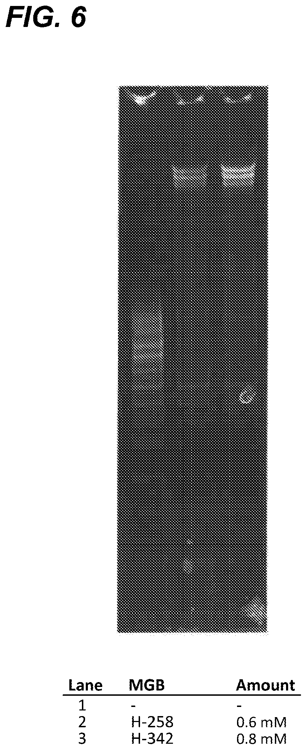

Using the primer extension reaction described in Example 1, titration experiments were performed to determine the optimal amount of MGB for enhancement of XNTP polymerization. Results from a representative experiment are presented in FIG. 5. These results demonstrate a dose-dependent enhancement with each MGB tested (Hoescht 33258, Hoescht 33342, and Netropsin, ranging from 75 to 600 µM) with the highest concentration showing the most robust enhancement of primer extension. Further titration experiments indicated that 0.6 mM Hoescht 33258 and 0.8 mM Hoescht 33342 demonstrate reliable enhancement of XNTP polymerization. A representative gel showing these effects is presented in FIG. 6.

### Example 3

### MGBs Enhance Sequencing by Expansion (SBX)

To investigate the accuracy of enhancement of XNTP polymerization, primer extension products were sequenced using the SBX protocol. Briefly, the constrained Xpandomer products of XNTP polymerization are cleaved to generate linearized Xpandomers. This is accomplished by first quenching the extension reaction with a solution containing 100 mM EDTA, 2 mM THPTA, and 2% Tween-20. Then the sample is subjected to amine modification with a solution of 1 M NaHCO₃ and 1 M succinic anhydride in DMF. Cleavage of the phosphoramidate bonds is carried out with 37% HCl and linearized Xpandomers are purified with QIAquick columns (QIAGEN, Inc.).

For sequencing, protein nanopores are prepared by inserting α-hemolysin into a DPhPE/hexadecane bilayer member in buffer B1, containing 2 M NH₄Cl and 100 mM HEPES, pH 7.4. The cis well is perfused with buffer B2, containing 0.4 M NH₄Cl, 0.6 M GuCl, and 100 mM HEPES, pH 7.4. The Xpandomer sample is heated to 70° C for 2 minutes, cooled completely, then a 2 µL sample is added to the cis well. A voltage pulse of 90mV/390mV/10µs is then applied and data is acquired via Labview acquisition software.

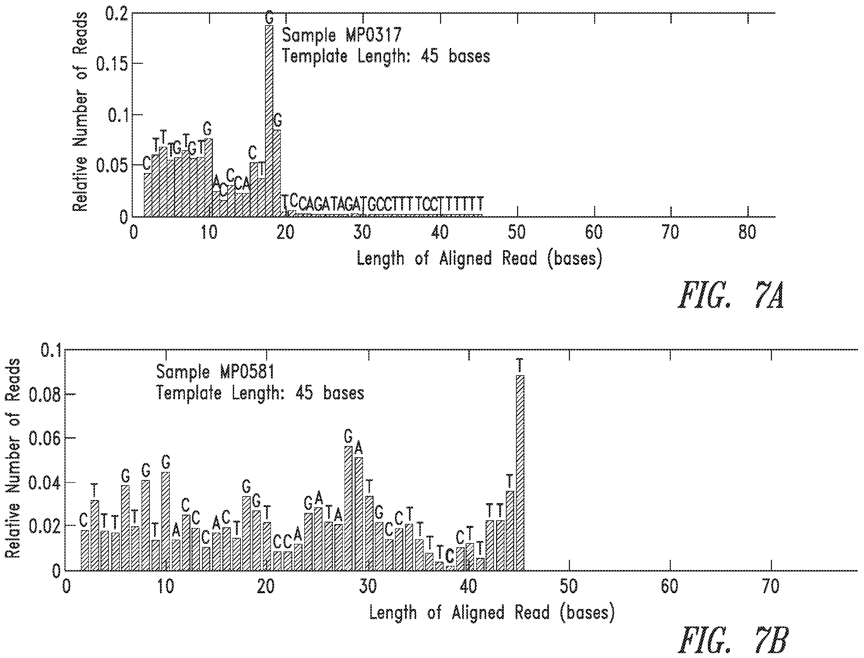

Sequence data was analyzed by histogram display of the population of sequence reads from a single SBX reaction. The analysis software aligns each sequence read to the sequence of the template and trims the extent of the sequence at the end of the reads that does not align with the correct template sequence. Representative histograms of SBX sequencing of a 45mer template are presented in FIG. 7A (no additive control) and FIG. 7B (SBX in the presence of Hoescht 33258). As can be seen, in the absence of additive, sequence reads are not accurate past base 20 of the template. Remarkably, addition of the MGB to the SBX reaction increased the accuracy of the sequence reads completely to the end of the 45mer template.

All of the U.S. patents, U.S. patent application publications, U.S. patent applications, foreign patents, foreign patent applications and non-patent publications referred to in this specification and/or listed in the Application Data Sheet, including but not limited to, U.S. Provisional Patent Application No. 62/614,114, refer to are materials and methodologies, which might be used in connection with the presently described invention. The publications discussed above and throughout the text are provided solely for their disclosure. Nothing herein is to be construed as an admission that the inventors are not entitled to antedate any referenced publication by virtue of prior invention.

In general, in the following claims, the terms used should not be construed to limit the claims to the specific embodiments disclosed in the specification and the claims, but should be construed to include all possible embodiments along with the full scope of equivalents to which such claims are entitled.

## Claims

1. A method of enhancing a nucleic acid polymerase reaction, the method comprising:
a) forming a nucleic acid polymerase reaction composition comprising:
(i) a template nucleic acid,
(ii) a nucleic acid polymerase,
(iii) a mixture of nucleotides or nucleotide analogs,
(iv) at least one minor groove binding moiety; and
b) incubating the nucleic acid polymerase reaction composition under conditions allowing a nucleic acid polymerization reaction, wherein the at least one minor groove binding moiety increases the processivity, rate, or fidelity of the nucleic acid polymerase reaction, wherein the nucleic acid polymerase is a DNA polymerase, and wherein the mixture of nucleotides or nucleotide analogs comprises nucleoside triphosphoramidates, wherein each of the nucleoside triphosphoramidates comprises a nucleobase selected from the group consisting of adenine, guanine, thymine, and cytosine and a polymeric tether element, wherein a first end of the polymeric tether element is attached to the nucleobase and a second end of the polymeric tether element is attached to the alpha phosphate of the nucleoside triphosphoramidate to provide for expansion of the nucleotide analogs by cleavage of the phosphoramidate bond.

2. The method of claim 1, wherein the at least one minor groove binding moiety increases the length of a resulting nucleic acid product compared to a nucleic acid polymerase reaction lacking the minor groove binding moiety.

3. A composition for enhancing the processivity, fidelity, or rate of a DNA polymerase reaction comprising at least one minor groove binding moiety and a mixture of nucleotide analogs, wherein the mixture of nucleotides or nucleotide analogs comprises nucleoside triphosphoramidates, wherein each of the nucleoside triphosphoramidates comprises a nucleobase selected from the group consisting of adenine, guanine, thymine, and cytosine and a polymeric tether moiety, wherein a first end of the polymeric tether moiety is attached to the nucleobase and a second end of the polymeric tether moiety is attached to the alpha phosphate of the nucleoside triphosphoramidate to provide for expansion of the nucleotide analogs by cleavage of the phosphoramidate bond.

4. The method of claim 1 or the composition of claim 3, wherein the at least one minor groove binding moiety comprises a plurality of minor groove binding moieties, preferably the plurality of minor groove binding moieties comprises minor groove binding moieties of different structural classes.

5. The method of claim 1 or the composition of claim 3, wherein the reaction composition or the composition further comprises a DNA intercalating agent.

6. The method of claim 1 or the composition of claim 3, wherein the reaction composition or the composition further comprises a polyanion recognition moiety.

7. The method of claim 1 or the composition of claim 3, wherein the mixture of nucleotide analogs comprises nucleotide analogs comprising a detectable label, preferably the detectable label is an optically detectable label selected from the group consisting of luminescent, chemiluminescent, fluorescent, fluorogenic, chromophoric or chromogenic labels.

8. A method of sequencing a DNA template, the method comprising the steps of:
a) forming a DNA polymerase reaction composition comprising:
(i) the DNA template,
(ii) a replication primer that complexes with the template,
(iii) a DNA polymerase,
(iv) a mixture of nucleotides or nucleotide analogs,
(v) at least one minor groove binding moiety,
b) incubating the DNA polymerase reaction composition under conditions allowing a DNA polymerization reaction, wherein the at least one minor groove binding moiety increases the rate, fidelity or processivity of the DNA polymerase reaction; and
c) determining the sequence of the nucleotides or nucleotide analogs in the resulting polymer of nucleotides or nucleotide analogs,
wherein the mixture of nucleotides or nucleotide analogs comprises nucleoside triphosphoramidates, wherein each of the nucleoside triphosphoramidates comprises a nucleobase selected from the group consisting of adenine, guanine, thymine, and cytosine and a polymeric tether element, wherein a first end of the polymeric tether element is attached to the nucleobase and a second end of the polymeric tether element is attached to the alpha phosphate of the nucleoside triphosphoramidate to provide for expansion of the nucleotide analogs by cleavage of the phosphoramidate bond.

9. The method of claim 1 or 8, or the composition of claim 3, wherein the at least one minor groove binding moiety is selected from the group consisting of distamycin A and synthetic analogs thereof, netropsin, (+)-CC-1065, duocarmycins, pyrrolobenzodiazepines, trabectin and analogs thereof, Hoechst dyes and derivatives thereof, lexitropsin, thiazotropsin A, diamidines, and polyamides, preferably the minor groove binding moiety is a Hoechst dye.

10. The composition of claim 3, further comprising a buffer comprising Tris OAc, NH₄OAc, PEG, DMF, polyphosphate 60, and MnCl₂.

11. The method of enhancing a nucleic acid polymerase reaction according to claim 1, wherein the nucleic acid polymerization reaction produces an expandable polymer of nucleotide analogs, wherein the expandable polymer encodes the nucleobase sequence information of the template nucleic acid.

12. The method of claim 11, wherein the conditions for allowing a nucleic acid polymerization reaction comprise a suitable polymerization buffer and an oligonucleotide primer.

13. The method of claim 12, wherein the suitable buffer comprises Tris OAc, NH₄OAc, PEG, DMF, polyphosphate 60, and MnCl₂.

14. The method of claim 1 or 8, wherein the DNA polymerase is DPO4 or a variant thereof.

15. The method of claim 8, wherein the resulting polymer of nucleotide analogs is an expandable polymer.

16. The method of claim 15, further including the step of contacting the expandable polymer with a phosphoramidate cleavage agent to produce an expanded polymer of nucleotide analogs.

17. The method of claim 16, wherein the polymeric tether element of each of the nucleotide analogs comprises a reporter moiety unique to the nucleobase of the analog.

18. The method of claim 17, wherein the reporter moieties produce a characteristic electronic signal.

19. The method of claim 18, wherein the step of determining the sequence of the nucleotide analogs comprises the step of translocating the expanded polymer of nucleotide analogs through a nanopore.

## Patentansprüche

1. Verfahren zum Steigern einer Nukleinsäurepolymerasereaktion, wobei das Verfahren Folgendes umfasst:
a) Bilden einer Nukleinsäurepolymerasereaktionszusammensetzung, umfassend:
(i) eine Matrizennukleinsäure,
(ii) eine Nukleinsäurepolymerase,
(iii) ein Gemisch aus Nukleotiden oder Nukleotidanaloga,
(iv) mindestens eine Minor-Groove-Bindeeinheit, und
b) Inkubieren der Nukleinsäurepolymerasereaktionszusammensetzung unter Bedingungen, die eine Nukleinsäurepolymerisationsreaktion ermöglichen, wobei die mindestens eine Minor-Groove-Bindeeinheit die Prozessivität, Geschwindigkeit oder Genauigkeit der Nukleinsäurepolymerasereaktion erhöht, wobei die Nukleinsäurepolymerase eine DNA-Polymerase ist und wobei das Gemisch aus Nukleotiden oder Nukleotidanaloga Nukleosidtriphosphoramidate umfasst, wobei jedes der Nukleosidtriphosphoramidate eine Nukleobase, die aus der Gruppe ausgewählt ist, die aus Adenin, Guanin, Thymin und Cytosin besteht, und ein polymeres Tetherelement umfasst, wobei ein erstes Ende des polymeren Tetherelements an die Nukleobase gebunden ist und ein zweites Ende des polymeren Tetherelements an das Alpha-Phosphat des Nukleosidtriphosphoramidats gebunden ist, um durch Aufspaltung der Phosphoramidatbindung Expansion der Nukleotidanaloga bereitzustellen.

2. Verfahren nach Anspruch 1, wobei die mindestens eine Minor-Groove-Bindeeinheit die Länge eines resultierenden Nukleinsäureproduktes im Vergleich zu einer Nukleinsäurepolymerasereaktion, der die Minor-Groove-Bindeeinheit fehlt, erhöht.

3. Zusammensetzung zum Steigern der Prozessivität, Genauigkeit oder Geschwindigkeit einer DNA-Polymerasereaktion, umfassend mindestens eine Minor-Groove-Bindeeinheit und ein Gemisch aus Nukleotidanaloga, wobei das Gemisch aus Nukleotiden oder Nukleotidanaloga Nukleosidtriphosphoramidate umfasst, wobei jedes der Nukleosidtriphosphoramidate eine Nukleobase, die aus der Gruppe ausgewählt ist, die aus Adenin, Guanin, Thymin und Cytosin besteht, und eine polymere Tethereinheit umfasst, wobei ein erstes Ende der polymeren Tethereinheit an die Nukleobase gebunden ist und ein zweites Ende der polymeren Tethereinheit an das Alpha-Phosphat des Nukleosidtriphosphoramidats gebunden ist, um durch Aufspaltung der Phosphoramidatbindung Expansion der Nukleotidanaloga bereitzustellen.

4. Verfahren nach Anspruch 1 oder Zusammensetzung nach Anspruch 3, wobei die mindestens eine Minor-Groove-Bindeeinheit eine Vielzahl von Minor-Groove-Bindeeinheiten umfasst, wobei vorzugsweise die Vielzahl von Minor-Groove-Bindeeinheiten Minor-Groove-Bindeeinheiten aus verschiedenen Strukturklassen umfasst.

5. Verfahren nach Anspruch 1 oder Zusammensetzung nach Anspruch 3, wobei die Reaktionszusammensetzung oder die Zusammensetzung ferner ein DNAinterkalierendes Mittel umfasst.

6. Verfahren nach Anspruch 1 oder Zusammensetzung nach Anspruch 3, wobei die Reaktionszusammensetzung oder die Zusammensetzung ferner eine Polyanionenerkennungseinheit umfasst.

7. Verfahren nach Anspruch 1 oder Zusammensetzung nach Anspruch 3, wobei das Gemisch aus Nukleotidanaloga Nukleotidanaloga umfasst, die eine nachweisbare Markierung umfassen, wobei vorzugsweise die nachweisbare Markierung eine optisch nachweisbare Markierung ist, die aus der Gruppe ausgewählt ist, die aus lumineszierenden, chemilumineszierenden, fluoreszierenden, fluorogenen, chromophoren oder chromogenen Markierungen besteht.

8. Verfahren zum Sequenzieren einer DNA-Matrize, wobei das Verfahren die folgenden Schritte umfasst:
a) Bilden einer DNA-Polymerasereaktionszusammensetzung, umfassend:
(i) die DNA-Matrize,
(ii) einen Replikationsprimer, der mit der Matrize komplexiert,
(iii) eine DNA-Polymerase,
(iv) ein Gemisch aus Nukleotiden oder Nukleotidanaloga,
(v) mindestens eine Minor-Groove-Bindeeinheit,
b) Inkubieren der DNA-Polymerasereaktionszusammensetzung unter Bedingungen, die eine DNA-Polymerisationsreaktion ermöglichen, wobei die mindestens eine Minor-Groove-Bindeeinheit die Geschwindigkeit, Genauigkeit oder Prozessivität der DNA-Polymerasereaktion erhöht, und
c) Bestimmen der Sequenz der Nukleotide oder Nukleotidanaloga in dem resultierenden Polymer von Nukleotiden oder Nukleotidanaloga,
wobei das Gemisch aus Nukleotiden oder Nukleotidanaloga Nukleosidtriphosphoramidate umfasst, wobei jedes der Nukleosidtriphosphoramidate eine Nukleobase, die aus der Gruppe ausgewählt ist, die aus Adenin, Guanin, Thymin und Cytosin besteht, und ein polymeres Tetherelement umfasst, wobei ein erstes Ende des polymeren Tetherelements an die Nukleobase gebunden ist und ein zweites Ende des polymeren Tetherelements an das Alpha-Phosphat des Nukleosidtriphosphoramidats gebunden ist, um durch Aufspaltung der Phosphoramidatbindung Expansion der Nukleotidanaloga bereitzustellen.

9. Verfahren nach Anspruch 1 oder 8 oder Zusammensetzung nach Anspruch 3, wobei die mindestens eine Minor-Groove-Bindeeinheit aus der Gruppe ausgewählt ist, die aus Distamycin A und synthetischen Analoga davon, Netropsin, (+)-CC-1065, Duocarmycinen, Pyrrolobenzodiazepinen, Trabectin und Analoga davon, Hoechst-Farbstoffen und Derivaten davon, Lexitropsin, Thiazotropsin A, Diamidinen und Polyamiden besteht, wobei vorzugsweise die Minor-Groove-Bindeeinheit ein Hoechst-Farbstoff ist.

10. Zusammensetzung nach Anspruch 3, ferner umfassend einen Puffer, der Tris OAc, NH₄OAc, PEG, DMF, Polyphosphat 60 und MnCl₂ umfasst.

11. Verfahren zum Steigern einer Nukleinsäurepolymerasereaktion nach Anspruch 1, wobei die Nukleinsäurepolymerisationsreaktion ein expandierbares Polymer aus Nukleotidanaloga produziert, wobei das expandierbare Polymer die Nukleobasensequenzinformationen der Matrizennukleinsäure kodiert.

12. Verfahren nach Anspruch 11, wobei die Bedingungen, die eine Nukleinsäurepolymerisationsreaktion ermöglichen, einen geeigneten Polymerisationspuffer und einen Oligonukleotidprimer umfassen.

13. Verfahren nach Anspruch 12, wobei der geeignete Puffer Tris OAc, NH₄OAc, PEG, DMF, Polyphosphat 60 und MnCl₂ umfasst.

14. Verfahren nach Anspruch 1 oder 8, wobei die DNA-Polymerase DPO4 oder eine Variante davon ist.

15. Verfahren nach Anspruch 8, wobei das resultierende Polymer aus Nukleotidanaloga ein expandierbares Polymer ist.

16. Verfahren nach Anspruch 15, das ferner den Schritt des Inkontaktbringens des expandierbaren Polymers mit einem Phosphoramidatspaltmittel unter Produktion eines expandierbaren Polymers aus Nukleotidanaloga einschließt.

17. Verfahren nach Anspruch 16, wobei das polymere Tetherelement von jedem der Nukleotidanaloga eine Reportereinheit umfasst, die für die Nukleobase des Analogons einzigartig ist.

18. Verfahren nach Anspruch 17, wobei die Reportereinheiten ein charakteristisches elektronisches Signal produzieren.

19. Verfahren nach Anspruch 18, wobei der Schritt des Bestimmens der Sequenz der Nukleotidanaloga den Schritt des Translozierens des expandierten Polymers aus Nukleotidanaloga durch eine Nanopore umfasst.

## Revendications

1. Procédé d'amélioration d'une réaction d'acide nucléique polymérase, le procédé comprenant :
a) la formation d'une composition de réaction d'acide nucléique polymérase comprenant :
(i) un acide nucléique modèle,
(ii) une acide nucléique polymérase,
(iii) un mélange de nucléotides ou d'analogues de nucléotides,
(iv) au moins une fraction de liaison à un petit sillon ; et
b) l'incubation de la composition de réaction d'acide nucléique polymérase dans des conditions permettant une réaction de polymérisation d'acides nucléiques, dans lequel l'au moins une fraction de liaison à un petit sillon augmente la processivité, la vitesse ou la fidélité de la réaction d'acide nucléique polymérase, dans lequel l'd'acide nucléique polymérase est une ADN polymérase, et dans lequel le mélange de nucléotides ou d'analogues de nucléotides comprend des nucléoside triphosphoramidates, dans lequel chacun des nucléoside triphosphoramidates comprend une nucléobase choisie dans le groupe constitué par l'adénine, la guanine, la thymine et la cytosine et un élément d'attache polymère, dans lequel une première extrémité de l'élément d'attache polymère est liée à la nucléobase et une seconde extrémité de l'élément d'attache polymère est liée au phosphate en alpha du nucléoside triphosphoramidate pour permettre l'expansion des analogues de nucléotides par clivage de la liaison phosphoramidate.

2. Procédé selon la revendication 1, dans lequel l'au moins une fraction de liaison à un petit sillon augmente la longueur d'un produit d'acide nucléique résultant par comparaison à une réaction d'acide nucléique polymérase dépourvue de la fraction de liaison à un petit sillon.

3. Composition permettant d'améliorer la processivité, la fidélité ou la vitesse d'une réaction d'ADN polymérase comprenant au moins une fraction de liaison à un petit sillon et un mélange d'analogues de nucléotides, dans laquelle le mélange de nucléotides ou d'analogues de nucléotides comprend des nucléoside triphosphoramidates, dans laquelle chacun des nucléoside triphosphoramidates comprend une nucléobase choisie dans le groupe constitué par l'adénine, la guanine, la thymine et la cytosine et une fraction d'attache polymère, dans laquelle une première extrémité de la fraction d'attache polymère est liée à la nucléobase et une seconde extrémité de la fraction d'attache polymère est liée au phosphate en alpha du nucléoside triphosphoramidate pour permettre l'expansion des analogues de nucléotides par clivage de la liaison phosphoramidate.

4. Procédé selon la revendication 1 ou composition selon la revendication 3, dans lesquels l'au moins une fraction de liaison à un petit sillon comprend une pluralité de fractions de liaison à un petit sillon, de préférence la pluralité de fractions de liaison à un petit sillon comprend des fractions de liaison à un petit sillon de différentes classes structurales.

5. Procédé selon la revendication 1 ou composition selon la revendication 3, dans lesquels la composition de réaction ou la composition comprend en outre un agent intercalant d'ADN.

6. Procédé selon la revendication 1 ou composition selon la revendication 3, dans lesquels la composition de réaction ou la composition comprend en outre une fraction de reconnaissance de polyanion.

7. Procédé selon la revendication 1 ou composition selon la revendication 3, dans lesquels le mélange d'analogues de nucléotides comprend des analogues de nucléotides comprenant un marqueur détectable, de préférence le marqueur détectable est un marqueur optiquement détectable choisi dans le groupe constitué par les marqueurs luminescents, chimioluminescents, fluorescents, fluorogènes, chromophores ou chromogènes.

8. Procédé de séquençage d'un modèle d'ADN, le procédé comprenant les étapes de :
a) formation d'une composition de réaction d'ADN polymérase comprenant :
(i) le modèle d'ADN,
(ii) une amorce de réplication qui se complexe avec le modèle,
(iii) une ADN polymérase,
(iv) un mélange de nucléotides ou d'analogues de nucléotides,
(v) au moins une fraction de liaison à un petit sillon,
b) incubation de la composition de réaction d'ADN polymérase dans des conditions permettant une réaction de polymérisation de l'ADN, dans lequel l'au moins une fraction de liaison à un petit sillon augmente la vitesse, la fidélité ou la processivité de la réaction d'ADN polymérase ; et
c) détermination de la séquence de nucléotides ou d'analogues de nucléotides dans le polymère de nucléotides ou d'analogues de nucléotides résultant,
dans lequel le mélange de nucléotides ou d'analogues de nucléotides comprend des nucléoside triphosphoramidates, dans lequel chacun des nucléoside triphosphoramidates comprend une nucléobase choisie dans le groupe constitué par l'adénine, la guanine, la thymine et la cytosine et un élément d'attache polymère, dans lequel une première extrémité de l'élément d'attache polymère est liée à la nucléobase et une seconde extrémité de l'élément d'attache polymère est liée au phosphate en alpha du nucléoside triphosphoramidate pour permettre l'expansion des analogues de nucléotides par clivage de la liaison phosphoramidate.

9. Procédé selon la revendication 1 ou 8, ou composition selon la revendication 3, dans lesquels l'au moins une fraction de liaison à un petit sillon est choisie dans le groupe constitué par la distamycine A et des analogues synthétiques de celle-ci, la nétropsine, le (+)-CC-1065, les duocarmycines, les pyrrolobenzodiazépines, la trabectine et des analogues de celle-ci, les colorants Hoechst et des dérivés de ceux-ci, la lexitropsine, la thiazotropsine A, les diamidines et les polyamides, de préférence la fraction de liaison à un petit sillon est un colorant Hoechst.

10. Composition selon la revendication 3, comprenant en outre un tampon comprenant Tris OAc, NH₄OAc, PEG, DMF, polyphosphate 60 et MnCl₂.

11. Procédé d'amélioration d'une réaction d'acide nucléique polymérase selon la revendication 1, dans lequel la réaction de polymérisation d'acides nucléiques produit un polymère d'analogues de nucléotides expansible, dans lequel le polymère expansible code les informations de la séquence de nucléobases de l'acide nucléique modèle.

12. Procédé selon la revendication 11, dans lequel les conditions permettant une réaction de polymérisation d'acides nucléiques comprennent un tampon de polymérisation approprié et une amorce oligonucléotidique.

13. Procédé selon la revendication 12, dans lequel le tampon approprié comprend Tris OAc, NH₄OAc, PEG, DMF, polyphosphate 60 et MnCl₂.

14. Procédé selon la revendication 1 ou 8, dans lequel l'ADN polymérase est DPO4 ou un variant de celle-ci.

15. Procédé selon la revendication 8, dans lequel le polymère d'analogues de nucléotides résultant est un polymère expansible.

16. Procédé selon la revendication 15, comprenant en outre l'étape de mise en contact du polymère expansible avec un agent de clivage de phosphoramidate pour produire un polymère d'analogues de nucléotides expansé.

17. Procédé selon la revendication 16, dans lequel l'élément d'attache polymère de chacun des analogues de nucléotides comprend une fraction rapporteur unique à la nucléobase de l'analogue.

18. Procédé selon la revendication 17, dans lequel les fractions rapporteurs produisent un signal électronique caractéristique.

19. Procédé selon la revendication 18, dans lequel l'étape de détermination de la séquence des analogues de nucléotides comprend l'étape de translocation du polymère d'analogues de nucléotides expansé à travers un nanopore.
